# EUROPEAN PATENT APPLICATION

(11) **EP 1 038 960 A1**
(43) Date of publication of application: **27.09.2000**
(21) Application number: 99400636.9
(22) Date of filing: 16.03.1999
(51) Int. Cl.: C12N 15/12, C07K 14/705, C12Q 1/68, C12N 15/85, C12N 5/10, C07K 16/28, G01N 33/53, G01N 33/68

(54) **BSMAP, a surface protein expressed specifically in the brain**

(71) Applicant: PIERRE FABRE MEDICAMENT, 92100 Boulogne (FR)
(72) Inventor: Elson, Greg, 78150 Saint Julien en Genevois (FR); Bonnefoy, Jean-Yves, 74350 Le Sappey (FR); Gauchat, Jean-François, 74580 Veigy (FR)
(74) Representative: Warcoin, Jacques

(57) **Abstract**

The present invention concerns a membrane-bound protein, BSMAP, which has been found to be both highly and preferentially expressed at the surface of cells in human brain. The invention is more particularly aimed at a polynucleotide encoding BSMAP, at the corresponding polypeptide, and at their use for raising antibodies as well as identifying agonist or antagonist compounds, which can be used for treating various CNS associated disorders and diseases.

## Description

### Field of the Invention

The present invention concerns a novel membrane-bound protein, BSMAP, which has been found to be both highly and preferentially expressed at the surface of cells in human brain. The invention is more particularly aimed at a polynucleotide encoding BSMAP, at the corresponding polypeptide, and at their use for raising antibodies as well as identifying agonist or antagonist compounds, which can be used for treating various CNS associated disorders and diseases.

### Background of the Invention

There is a great interest to search for receptors likely to be involved in psychiatric disorders, such as the dopamine and serotonin receptor subtypes, and also for various transporters. Several receptors are well known for being involved in disorders such as depression, dyslexia, eating disorders, epilepsy, migrane, panic disorder, schizophrenia, obsessive and compulsive disorders. D1- D2- and D3-dopamine receptors, the 5-HT1A, 5-HT1B/D, 5-HT2A and 5-HT4 receptors, and GABA receptors have been shown to be useful target by agonist and antagonist compounds to treat and/or prevent CNS disorders. Brain receptors in general are also attractive candidates for finding new therapies for brain diseases such as amotrophic lateral sclerosis, Alzheimer's disease, brain tumors, Huntington's disease, multiple sclerosis, Parkinson's disease and stroke. One strategy to identify new brain receptors consists in the finding of open reading frames and exons within the human genome. Sequence, located at sites that have been shown to be associated with CNS genetic phenotypes, may be involved in brain function. However, the quest for such sequences is impaired by the huge complexity of the genome and the lack of consistent pedigrees for a given disease.

Human chromosome 19 is a short chromosome with a relatively high GC content (Larsen et al., 1992), which has been found to be involved in CNS functions. It has a high gene density compared to other chromosomes with an abundance of Alu repeats (Korenberg and Rykowski, 1988). It has been well characterized at the genetic and molecular level (Ashworth et al., 1995), with a number of genes giving rise to the pathogenesis of various human diseases being mapped on this chromosome. These include amongst others the low density lipoprotein receptor (LDL-R) implicated in familial hypercholesterolemia (Francke et al., 1984), the erythropoietin receptor (EPOR) implicated in primary familial and congenital polycythemias (PFCP; (Sokol et al., 1995)) and apolipoprotein E (APOE) playing a role in late onset familial Alzheimer's disease (Strittmatter and Roses, 1995).

The gene for the type I cytokine receptor homologue CLF-1 (Elson et al., 1998) was recently localized to chromosome 19 using the sequence derived from a chromosome 19p12-specific cosmid R30292, which was sequenced at the Human Genome Center. Using the CLF-1 cDNA sequence to query the GenBank database, all nine CLF-1-specific exons were identified within the cosmid sequence.

Unexpectedly, seven other exons coding in the reverse orientation, located adjacent to the above mentionned CLF-1 exons, have been found. The present invention thus provides a new gene, located in chromosome 19, which has been named Brain-Specific Membrane Anchored Protein (BSMAP). This gene encodes a putative 40 kDa membrane-bound type-I glycoprotein whose mRNA is strongly and preferentially expressed in the CNS. Such highly preferential tissue expression of BSMAP mRNA shows that this membrane protein corresponds to a receptor implicated in brain function. BSMAP shows no significant homology with any known proteins. It shares some degree of homology with the amino acid sequence derived from a novel cDNA cloned from CD34⁺ hematopoietic progenitors (Mao et al, 1998). This cloned cDNA (HSPC001) has been assigned the accession number AF047439 and has homology with mouse tropomyosine, which prima facie has nothing to do with brain receptors. Furthermore, BSMAP mRNA tissue distribution is quite distinct from HSPC001.

Consequently, the above mentioned prior art documents do not describe, nor suggest the present invention as defined hereafter.

### DESCRIPTION

The present invention concerns a polynucleotide comprising a sequence having at least 50% identity with the sequence of SEQ ID n°1, coding for BSMAP, BSMAP is referred herein as the human BSMAP protein but also its mammalian counterparts such as the mouse BSMAP. Preferably, the polynucleotide is coding for the human BSMAP and has at least 80% identity with the sequence of SEQ ID n°1.
As used herein, the term "polynucleotide" refers to a natural or non natural nucleic acid, eventually chemically modified, or a fragment thereof, which is chosen from the group consisting of DNA, cDNA, and RNA. The invention is aimed at equivalent polynucleotide. The term "equivalent" means any polynucleotide coding for all or parts of the BSMAP polypeptide, taken into account the degeneracy of the genetic code, as well as any polynucleotide coding for equivalent BSMAP polypeptides.

Another aspect of the invention relates to an oligonucleotide capable of hybridizing under stringent conditions a polynucleotide as defined above for a use as a probe or a primer. Advantageously, this oligonucleotide comprises at least 8 consecutive bases of a polynucleotide according to the invention for a use as a probe or a primer. An oligonucleotide which is a complementary antisense oligonucleotide to a sequence coding for BSMAP as well as an oligonucleotide which is labeled with a fluorescent or radioactive tag are especially intended as a preferred embodiment. The above oligonucleotides are useful for the detection and/or the amplification of a sequence coding for BSMAP in a sample.
Another embodiment is a method for detecting a sequence coding for BSMAP in a sample consisting of hybridizing the DNA contained in said sample with a oligonucleotide according to the invention and analyzing the signal.
Such a method for detecting a sequence coding for BSMAP in a sample may comprise the steps consisting of :
a) Isolating and/or purifying the DNA extracted from a sample, or obtaining the cDNA corresponding to the RNA contained in a sample,
b) amplifying specifically said DNA or cDNA using oligonucleotides according to the invention,
c) hybridizing the products obtained in step b) with a labeled oligonucleotide as described above, and analyzing the signal.
Still another embodiment of the invention is a kit for the detection and/or the amplification of a sequence coding for BSMAP in a sample comprising at least one of said oligonucleotides and reagents necessary for detecting and/or amplifying.

Another aspect of the invention is aimed at a recombinant vector comprising a polynucleotide as defined above for the cloning and/or the expression of said polynucleotide.
This vector may comprise at least one element allowing the efficient expression of said polynucleotide or fragment of polynucleotide in a host cell.
The invention is also directed to a host cell transformed by said vector and to a process for preparing a BSMAP polypeptide, or fragment thereof, wherein said host cells are cultured in conditions allowing an efficient production of said polypeptide, and also to a process for preparing a BSMAP polypeptide using said vector.

A further aspect of the invention relates to a polypeptide obtainable by a process as described above. A polypeptide comprising an amino acid sequence having at least 70 % identity with the sequence of SEQ ID n°2 is another embodiment. Said polypeptide is chosen from the group consisting of a homologue, a derivative, an equivalent, and a fragment of a polypeptide.
As used herein, the term "equivalent" will be understood to mean a peptide having at least one of the activities of the instant polypeptide. "Homologue" will be understood as a polypeptide exhibiting certain modifications compared with the natural polypeptide. These modifications can be a deletion, a truncation, an extension, a chimeric fusion, and/or a mutation. Among equivalent polypeptides, those who display more than 80% homology are preferred.

"Derivative" refers to any polypeptides, eventually mutated, truncated, and/or extended, which have been chemically modified or contain unusual amino acids.

Another aspect of the invention relates to a monoclonal or polyclonal antibody which is capable of binding selectively to a polypeptide as defined above. Said monoclonal antibody may be capable of modulating BSMAP activity. Antibodies may also be labeled with a radioactive or a fluorescent tag, or linked to an enzyme reacting with a chromogene, a fluorigene or luminescent substrate.

The term "tag" as used herein, whether it is intended to label a nucleic acid or an antibody, it to be understood as any molecules that can be detected using conventional methods or that induces the detection of other molecules. Among such tags, those who are preferred are radioactive isotopes, compounds containing an isotope, enzymes, in particular enzymes susceptible to react with chromogenes, fluorigenes or luminescents (for example a peroxydase or an alcaline phosphatase), chromophores, chromogene compounds , fluorigenes ou luminescents, base analogues , and ligands such as biotin.

Another embodiment concerns a kit for detecting a polypeptide according to the invention in a sample comprising a monoclonal or polyclonal antibody as defined above, a buffer favoring the polypeptide-antibody complex formation, and optionally agents allowing the detection of said complex.

Still another aspect of the present invention relates to a process for screening agonist or antagonist compounds comprising the step consisting of testing the affinity and/or the effect of said compounds on a polypeptide according to the invention. Another possibility lies in the testing of said compounds on a host cell according to the invention.

The present invention is also aimed at anagonist or antagonist compound obtainable from said process and to any compound that modulates BSMAP activity. Such compounds may be selected from chemicals, antibodies, peptides, and peptide derivatives.
The invention also concerns a pharmaceutical composition comprising said compound and a pharmaceutically acceptable carrier.

Another aspect of the invention relates to a method for treating and/or preventing CNS disorders comprising the step of administering a pharmaceutically effective amount of a compound as described above.

Among CNS disorders, those who are the most aimed are depression, dyslexia, dystonia, eating disorders, epilepsy, migrane, headache, panic disorder, schizophrenia, obsessive and compulsive disorders.

The present invention also concerns a method for treating and/or preventing CNS diseases comprising the step of administering a pharmaceutically effective amount of said compound.
Among CNS diseases, those who are the most aimed are amotrophic lateral sclerosis, multiple sclerosis, Alzheimer's disease, brain tumors, Huntington's disease, Parkinson's disease and stroke.

The detailed description will appear more clearly by referring to the figure legends presented below.

### LEGENDS

### Figure 1: Nucleic acid and deduced amino acid sequence of exalt 1 of the gene encoding BSMAP.

The black arrow represents the predicted splice donor while the gray arrow represents the predicted splice acceptor. The hypothetical BSMAP amino acid sequence including the predicted start methionine is shown in bold type. Numbers shown represent bases from the start of the cosmid R30292.

### Figure 2: Genomic organization of the genes encoding CLF-1 and BSMAP.

Gray boxes represent exons which are numbered. A partial restriction map covering the two genes is shown above the exons. The line below represents the assembly of the cosmids R30292 and R30085, with numbers representing the distance in bases from the start of the cosmid R30292.

### Figure 3: Detection of BSMAP mRNA expression in a panel of human cells and cell lines.

Poly A⁺ RNA was extracted from the cells indicated and subjected to RT-PCR using primers specific for the BSMAP cDNA sequence. As a positive control for RNA preparation and reverse transcription, PCR was performed with primers specific for the β-actin cDNA sequence. Products were analyzed on a 1.5 % agarose gel.

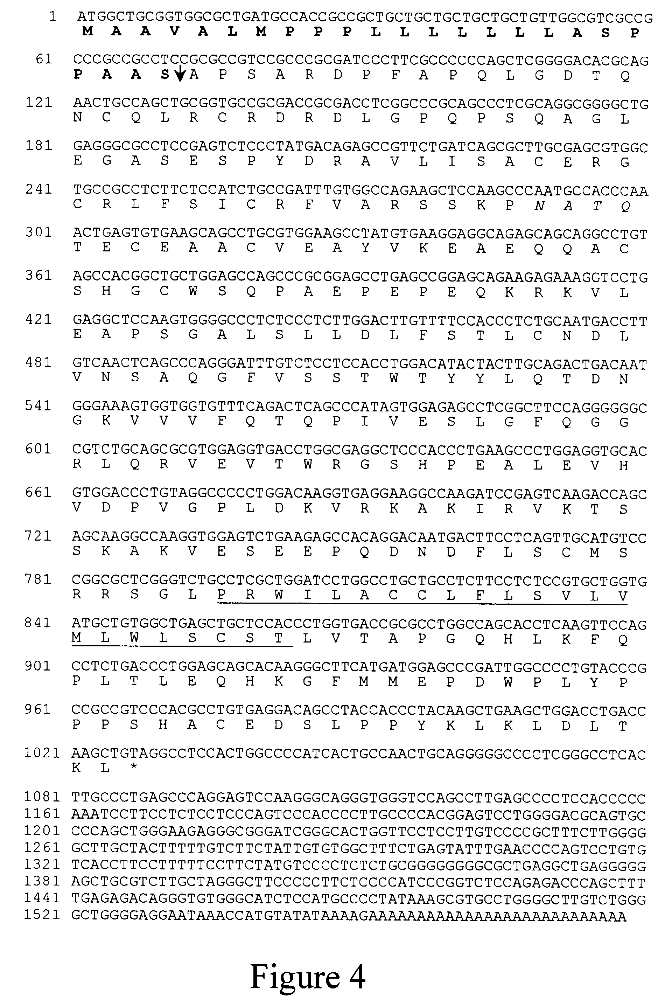

### Figure 4: Nucleotide and deduced amino acid sequence of the BSMAP cDNA.

An open reading frame encodes a polypeptide consisting of 342 amino acids. The putative signal peptide is shown in bold with the black arrow indicating the potential cleavage site. The potential N-linked glycosylation site is shown in italic. The putative transmembrane domain is underlined.

### Figure 5A:

Alignment of the BSMAP amino acid sequence with the deduced amino acid sequence of the novel cDNA derived from CD34⁺ hematopoietic progenitors using CLUSTAL W.
Residues boxed in black are those showing amino acid similarity or identity, with a consensus sequence shown above the alignment.

### Figure 5B-5D:

Alignment of the BSMAP amino acid sequence with mouse (B and C) and rat (D) derived ESTs using CLUS'I'AL W. Residues are boxed as above, with a consensus sequence shown above the alignment. cDNAs are identified by their database accession number.

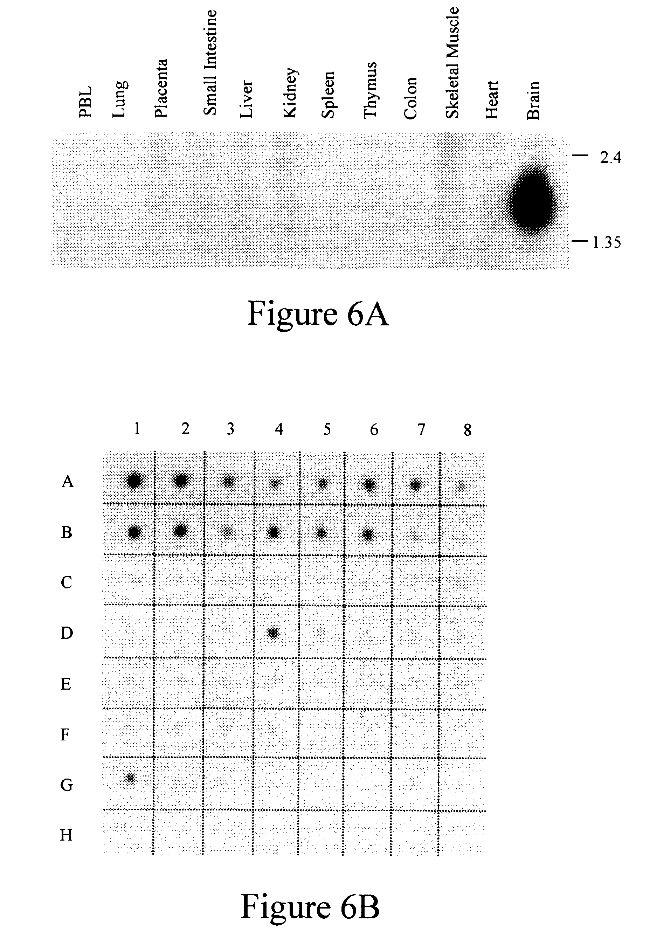

### Figure 6: Northern and Dot blot analysis of BSMAP mRNA tissue distribution.

Poly A⁺ mRNA blots were hybridized with a radiolabeled cDNA encoding the full length ORF as a probe.
**(A)** Northern blot containing poly A⁺ RNA derived from 12 different human tissues as indicated. Autoradiography exposure time was 8 hours at -70°C.
**(B)** Dot blot containing poly A⁺ RNA derived from 50 different human tissues. A1, whole brain; A2, amygdala; A3, caudate nucleus; A4, cerebellum; A5, cerebral cortex; A6, frontal lobe; A7, hippocampus; A8, medulla oblongata; B1, occipital lobe; B2, putamen; B3, substantia nigra; B4, temporal lobe; B5, thalamus, B6, nucleus accumbeus; B7, spinal cord; D4, pituitary gland, G1, fetal brain. Autoradiography time was 2 hours at room temperature.

### Figure 7: Detection of BSMAP in transfected COSI cells by western blotting.

Cells were transfected with cDNA constructs encoding BSMAP with or without a C-myc antibody recognition tag as indicated. Proteins from transfected cell lysates (15 µl) and supernatants (volume indicated) were resolved under reducing conditions on a 10 % SDS-polyacrylamide gel, electrotransferred to nitrocellulose filters and detected with an anti-C-myc tag mAb.

### Detailed Description

A gene, localized on chromosome 19p12, encoding a novel membrane-bound human protein (BSMAP) has been identified and its corresponding cDNA has been cloned. BSMAP specific mRNA was found to be both highly and preferentially expressed in human brain.

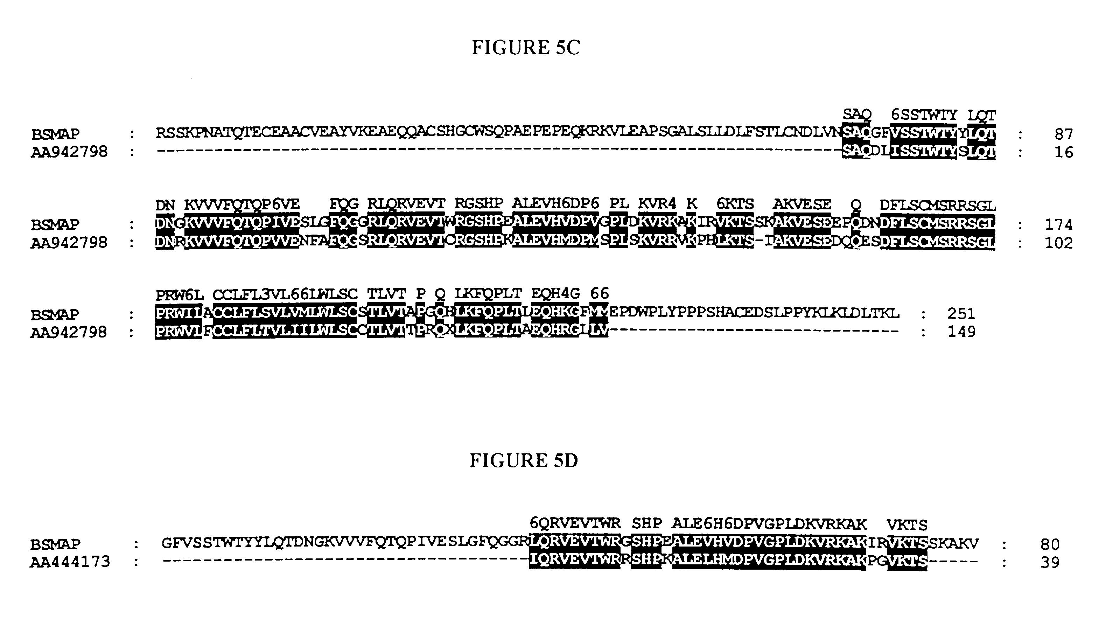

BSMAP shows no significant homology with any known proteins. It shares some degree of homology with the protein sequence derived from a novel and uncharacterized cDNA recently deposited in GenBank and cloned from CD34⁺ hematopoietic precursor cells (Mao et al., 1998). The homology extends throughout both protein sequences including a strong homology within the transmembrane domain of BSMAP. This suggests that this novel CD34⁺ precursor cell protein could also be a membrane-bound protein. The proposed start methionine did not, however precede a putative signal peptide. CD34' precursor cell cDNA sequence was used to query dbEST and identified a large number of ESTs encoding the novel protein and having an extremely wide ranging distribution, suggesting a fairly ubiquitous expression pattern. It therefore appears, judging by the source of origin of ESTs for BSMAP and the novel CD34⁺ precursor cell protein, that although these two proteins share some degree of homology (37%, see figure 5A), they do not share the same tissue distribution.

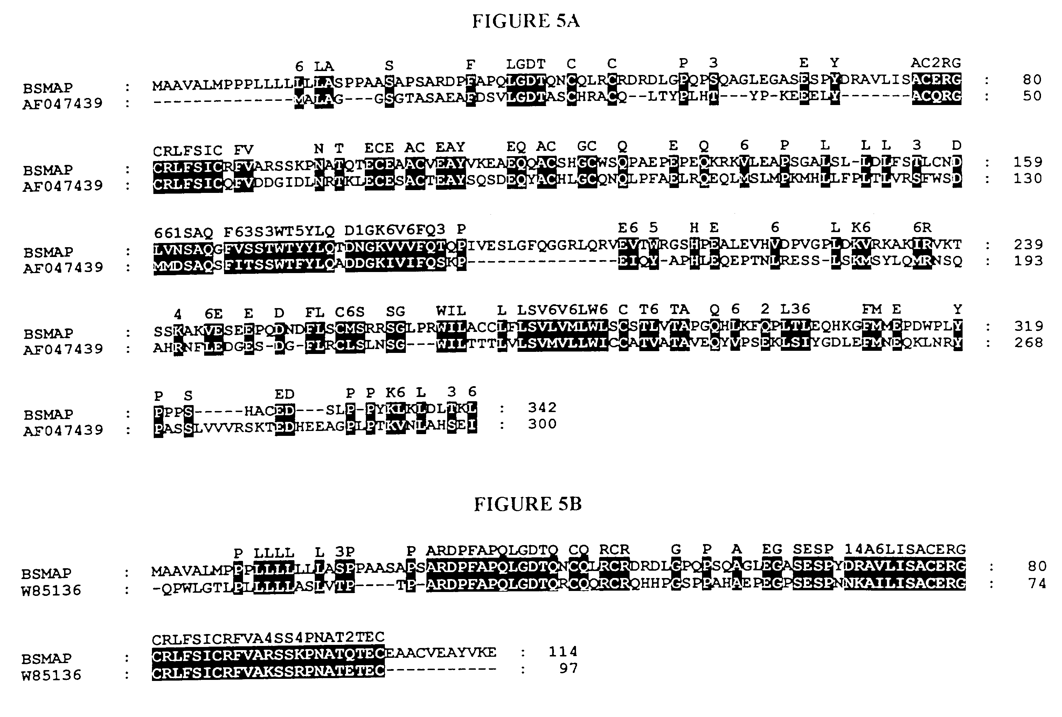

The BSMAP amino acid sequence has been used to identify two mouse derived ESTs and one rat derived EST. When translated, these ESTs share a significant level of homology with BSMAP (figure 5 B-D). Such a high level of homology (70 % and 82 % identity between BSMAP and the two mouse ESTs and 73 % identity between BSMAP and the rat EST) indicates that these ESTs were derived from cDNA clones encoding the mouse and rat homologues of BSMAP. This is further supported by the fact that the rat EST was sequenced from a cDNA clone derived from brain tissue (see table I below).

**Table I.**

| ESTs corresponding to BSMAP in dbEST | |
|---|---|
| Source of Origin | Number of BSMAP Specific ESTs |
| Human Brain | 34 |
| Human Fetus | 3 |
| Human Synovial Sarcoma | 3 |
| Human Genomic DNA | 2 |
| Rat Brain | 1 |
| Mouse Embryo | 2 |

BSMAP specific mRNA is preferentially expressed at high levels in brain tissue and in the pituitary gland. Northern blotting strongly revealed a single transcript of approximately 1.5 kb in brain following a short time of exposure (8 hours; Figure 6A). The relative expression of BSMAP mRNA in 15 different regions of the CNS, using a polyadenylated RNA dot blot, has therefore been assessed. The dot blot also contained RNA derived from 35 other human tissues, including total brain. A short time of exposure (2 hours) allowed the determination of small differences in the level of expression of BSMAP mRNA in the different regions of the brain. Signals could be detected in most of the CNS tissues, suggesting that BSMAP mRNA was expressed throughout the CNS and was not restricted to one particular area (Figure 6B). Strongest expression was seen in amygdala, occipital lobe and putamen. Thus, BSMAP mRNA expression is strong and preferential in the CNS.

Transient transfection in COS1 cells was performed in order to verify that the cloned cDNA encoded a protein product, and to determine its sub-cellular localization. A C-myc antibody recognition tag was inserted into the predicted extracellular region of the protein to allow recognition of the protein by western blotting, fluorescent cell staining and flow cytometry. By western blotting, a signal was only detected in the lysate of cells transfected with the BSMAP/C-myc expression construct and not in the supernatant, suggesting as predicted from the amino acid sequence that the protein was not soluble. A single band was seen with a size corresponding well with the predicted non-glycosylated protein size of 37.6 kDa. It should be noted that BSMAP has only one N-linked glycosylation site, thus it is unlikely that glycosylation causes a large increase in the molecular weight of the protein (Figure 7).

Fluorescent cell staining also revealed that the protein was well expressed. Due to its preferential tissue distribution, BSMAP, a type-I membrane-bound glycoprotein, is therefore a protein likely playing a role in the regulation of brain function.

It follows from the above results that BSMAP represents a good candidate receptor for the testing of compounds modulating its activity in the brain.

### EXAMPLE 1: Identification of a novel gene located adjacent to the gene encoding CLF-1

### Materials and Methods : Sequencing and sequence analysis

The sequence of the cosmid R30292 is deposited in the GenBank database with accession number AC003112. The sequence of the cosmid R30085 was obtained from the Lawrence Livermore national laboratory (LLNL) human genome center homepage on the Internet (http://bbrp.llnl.gov/sequence/bin/seqproject_contigs?proj=30085). Exons predicted within the cosmid were identified using GRAIL 2 (Uberbacher and Mural, 1991) and through sequence similarity with ESTs. cDNA sequencing was performed using the dideoxy chain termination method (Ausubel et al, 1991) with the T7 and SP6 primers, as well as specific primers designed according to the predicted BSMAP cDNA sequence. Sequencing reactions were performed using the AB1 Prism™ BigDye Terminator DNA sequencing kit (Perkin Elmer Applied Biosystems, Warrington, UK) following manufacturer's guidelines and analyzed on an ABI Prism™ 377 DNA sequencer (Perkin Elmer Applied Biosystems). Sequences obtained from cDNA clones as well as all relevant ESTs were imported into and analyzed by the Sequencher sequence analysis software (Genecodes, Ann Arbor, Ml). Nucleotide and predicted amino acid sequence homologies were identified using BLASTN, TBLASTN and BLASTP searches of GenBank and dbEST at the NCBI WWW server (http://www.ncbi.nlm.nih.gov/BLAST). Homologies were subsequently analyzed using the CLUSTALW program (Thompson et al., 1994). Signal peptide and transmembrane region predictions were performed using the SignalP (http:/www.cbs.dtu.dk/services/SignalP) and TMpred (http://www.isrec.isb-sib.ch/sotfware/ TMPRED_form.htm1) programs respectively.

### Results

Sequence analysis of the DNA located 3' to the gene for CLF-1 (Elson et al., 1998), within the 40668 bp cosmid R30292 derived from chromosome 19p12 between the UBA52 gene and the marker D195451, revealed seven putative exons in a reverse orientation to those encoding CLF-1. Six of these putative exons aligned with a number of different ESTs indicating that they were transcribed. The seven exons were subsequently assembled and the resulting sequence was used to query the GenBank database and dbEST, Although the sequence matched with no known cDNA sequences in the GenBank database, 42 ESTs of human origin were identified sharing a high degree of homology with the novel putative cDNA sequence, along with 2 ESTs of mouse origin and 1 of rat origin.

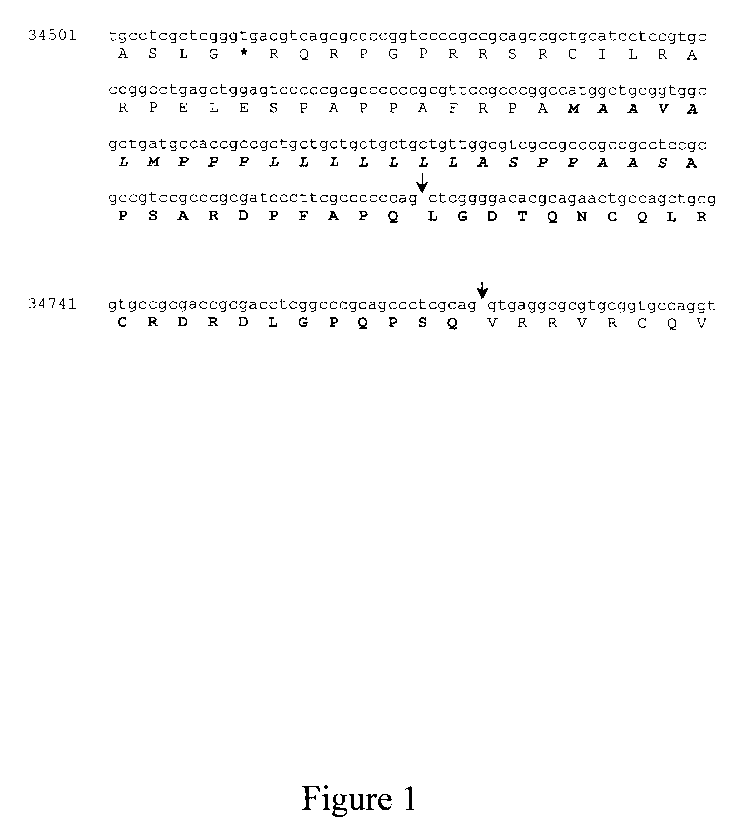

Computer aided alignment of the 42 human ESTs allowed the assembly of a 1364 bp contig with a 285 amino acid ORF and a 3' poly A tail. No putative ATG start codon was identified, suggesting that the cDNA sequence was incomplete. In addition, the amino acid sequence derived from the first predicted exon of the novel gene could be extended beyond the putative splice acceptor site at nucleotide 34712 to an ATG start codon starting at nucleotide 34607. This ATG codon was preceded by a Kozak consensus sequence (Kozak, 1989) and was followed by sequence which coded for a putative signal peptide in the same frame. It is therefore hypothesized that the exon was in fact larger than predicted and encodes a putative ATG start codon, which is further supported by the presence of an upstream TGA stop codon at nucleotide 34514 in frame with the ATG codon in question (Figure 1).

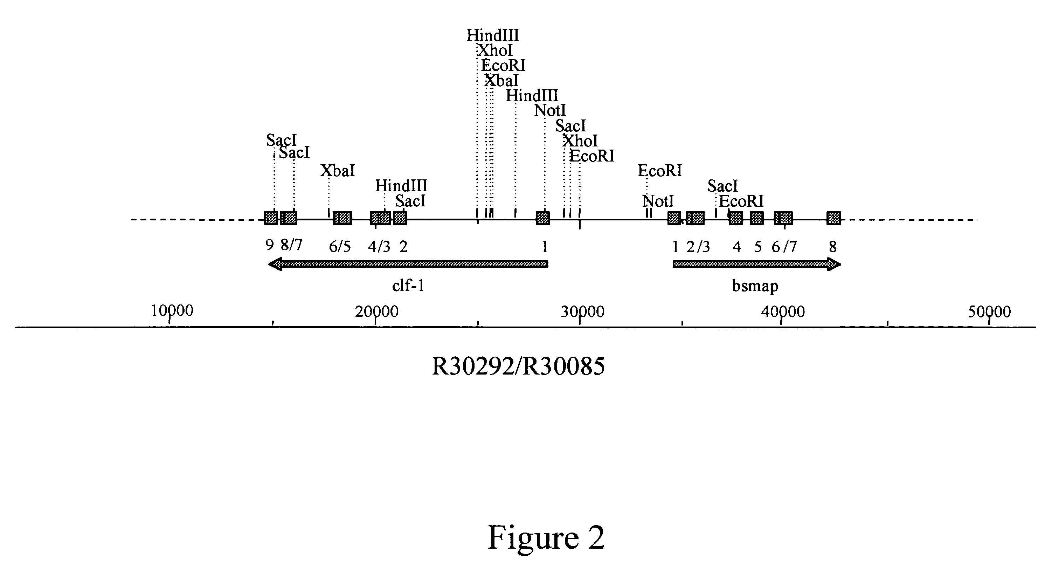

The fact that the last predicted exon (exon 7, running from nucleotides 40023 to 40140) aligned to nucleotides 612 to 729 of the EST derived cDNA contig suggested that only the partial gene sequence was present in the cosmid R30292. The 3' end of the R30292 cosmid sequence to identify a cosmid (R30085) overlapping with R30292 at the 3' end was therefore used. This additional sequence information allowed us to identify the last exon and therefore the complete gene structure. The novel gene is made up of 8 exons ands spans a region of 8080 bp (Figure 2 and Table II below).

**Table II.**

| Genomic Structure of the bsmap gene derived from the Cosmids R30292 and R30085. | | | | | | |
|---|---|---|---|---|---|---|
| Exon | Position # | Exon size | Intron | Intron size | 5' exon-intron junction | 3' intron-exon junction |
| 1 | x-171² | (x+171) | | | CGCAGgtgaggcg | TcccccagGCGGG |
| | | | 1 | 742 | | |
| 2 | 172-316 | 144 | | | AGCAGgtgagggc | TcccacagCCTGC |
| | | | 2 | 86 | | |
| 3 | 317-408 | 91 | | | AGAAGgtgggcct | TtcaccagAGAAA |
| | | | 3 | 1779 | | |
| 4 | 409-561 | 152 | | | TTCAGgtgagacc | TcjgcccagAcTcA |
| | | | 4 | 872 | | |
| 5 | 562-664 | 102 | | | CGTGGgtaaggtg | TctctcagACCCT |
| | | | 5 | 1052 | | |
| 6 | 665-782 | 117 | | | TCCCGgtgggtgg | CggggcagGCGCT |
| | | | 6 | 102 | | |
| 7 | 783-900 | 117 | | | TCCAGgtgggtgg | TcctgcagCCTCT |
| | | | 7 | 1914 | | |
| 8 | 901-1532 | 631 | | | - | - |

| | | | | | | |
|---|---|---|---|---|---|---|
| ² 'x' corresponds to the site of the start of transcription or the splice acceptor site for this particular exon. | | | | | | |

### EXAMPLE 2: Identification of cells expressing BSMAP specific mRNA.

### Materials and Methods:

### 1- Source of Cells and Culture Conditions

The fibroblastic cell lines HEK 293, COS1, and the B cell lines RPM1 8226 and RPMI 8866 were obtained from the American Type Culture Collection (ATCC; Manassas, VA) and cultured according to their specifications. The mast cell litre HMC-1 was maintained in Dulbecco's modified Eagle's medium and Ham's Fl2 nutrient mix supplemented with 10 % FCS, The source and culture conditions of the myeloma cell line U266, the B cell lymphoma HFB1 and the human T cell clone JF7 have been previously reported ((Pochon et al., 1992); (Gauchat et al., 1993)). Human umbilical vein endothelial cells (HUVEC) were isolated and maintained as previously described (Schnyder et al., 1996).

### 2- Detection of BSMAP mRNA by RT-PCR

Poly A⁺ RNA was isolated from the appropriate source as previously described (Okayama et al., 1987). cDNA was subsequently synthesized from 5 µg poly A⁺ RNA by random hexamer priming using SuperScript II reverse transcriptase (Life Technologies Ltd, Paisley, UK). 1/50^{th} of the cDNA was subsequently amplified by 30 cycles of PCR using the primers 5'-TGCCGCCTCTTCTCCATCTG-3'(SEQ ID n°3) and 5'-AGCCGAGGCTCTCCACTATG-3'(SEQ ID n°4) for BSMAP cDNA and 5'-GGCGACGAGGCCCAGAGCAAG-3'(SEQ ID n°5) and 5'-CGATTTCCCGCTCGGCCGTG-3'(SEQ ID n°6) for β-actin cDNA using the following conditions: 94°C for 30 seconds, 60°C for 30 seconds and 72°C for 1 minute. PCR products were analyzed by agarose gel electrophoresis.

### Results

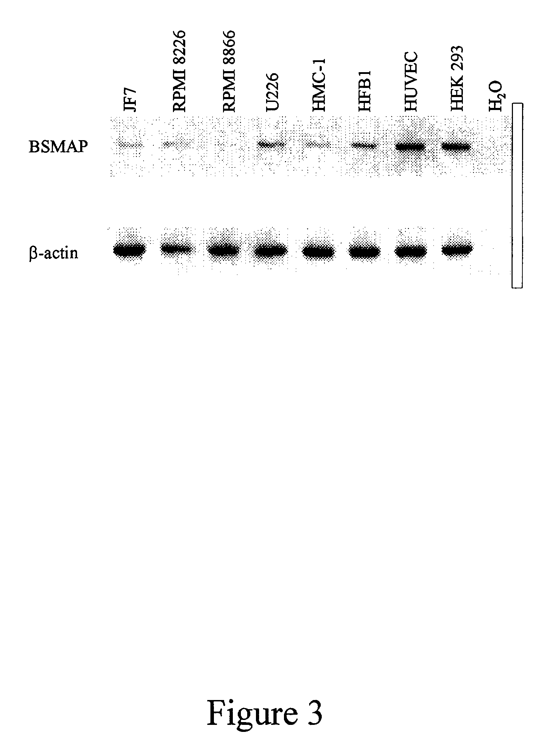

A panel of different human cells and cell lines was screened by RT-PCR for BSMAP mRNA expression, using primers located within the putative ORF and amplifying a cDNA product of 348 bp. As a control for the RT-PCR reaction, β-actin specific synthetic oligonucleotide primers were employed, amplifying a cDNA product of 462 bp.
BSMAP specific mRNA was detectable by RT-PCR in all cells and cell lines tested (Figure 3). Strongest signals were found with the HUVEC and the HEK 293 cell lines. The latter was therefore chosen to clone the BSMAP cDNA.

### EXAMPLE 3: BSMAP cDNA cloning.

### Materials and Methods

BSMAP cDNA fragments were amplified by anchored PCR using adapter-ligated double stranded cDNA prepared from HEK 293 cells. The cDNA was prepared using the Marathon cDNA amplification kit (Clontech Laboratories, Inc., Palo Alto, CA) following manufacturer's guidelines. For the PCR amplification of 5' cDNA ends, the BSMAP specific oligonucleotide primer with the sequence 5'-TTGGCCTTGCTGCTGGTCTT-3'(SEQ ID n°7) was used along with the adapter primer 1 (AP-1) provided in the Marathon cDNA amplification kit. For the PCR amplification of 3' cDNA ends, the AP-1 primer was used along with the BSMAP specific oligonuclcotide primer with the sequence 5'-TGCCGCCTCTTCTCCATCTG-3'(SEQ ID n°8). All PCR amplification conditions were as follows : an initial denaturation step of 94°C for 4 minutes, 35 cycles of 94°C for minute, 60°C for 1 minute, 72°C for 2 minutes and an elongation step of 72°C for 10 minutes. PCR products were cloned directly into the TA cloning vector pGENI-Teasy (Promega Corp., Madison, Wl) and resulting clones screened for a BSMAP-specific insert by colony hybridization using the DIG system for filter hybridization (Boehringer Mannheim, Mannheim, Germany) following manufacturer's guidelines, with a DIG-labeled oligonucleotide having the sequence 5'-TTGTCTCCTCCACCTGGACATACTAC-3'(SEQ ID n°9). BSMAP cDNA containing the full length ORF was amplified by PCR using the Advantage®-GC cDNA PCR kit (Clontech Laboratories, Inc.) with the primers 5'-ATGGCT GCGGTGGCGCTGAT-3' (SEQ ID n°10) and 5'-GGCCTACAGCTTGGTCAGGT-3'(SEQ ID n°11) following the manufacturer's guidelines. Amplified cDNA products of the appropriate size (1031 bp) were directly cloned into the TA cloning vector pGEM-Teasy (Promega Corp.).

### Results

BSMAP specific cDNA fragments were amplified from HEK 293 cell cDNA by anchored PCR through the rapid amplification of 5' and 3' cDNA ends (5' and 3' RACE). Subsequent alignment of the sequences derived from the cloned BSMAP-specific amplified fragments generated a 1516 bp contig with an ORF of 307 amino acids. The consensus sequence showed no nucleotide discrepancies with those derived from the *bsmap* gene exon assembly and the EST derived contig for BSMAP.

The BSMAP cDNA coding region was cloned by PCR amplification from HEK 293 derived first strand cDNA using BSMAP-specific synthetic oligonucleotides designed to amplify a PCR product of 1031 bp encoding the full length ORF, including the hypothetical ATG start codon. DMSO and a high fidelity proofreading DNA polymerase mix were included in the reaction in order to overcome the problems of high GC content and PCR induced errors, respectively. A DNA product of a size corresponding to that predicted for the BSMAP cDNA was amplified and subsequently cloned. Sequence analysis confirmed that the derived cDNA sequence contained the hypothetical ATG start codon, and contained no nucleotide mismatches when compared to the cDNA sequence derived from the predicted exon assembly and the assembled BSMAP cDNA sequence obtained by anchored PCR.

Analysis of the full length ORF revealed a protein sequence of 342 amino acids with one potential N-linked glycosylation site. Hydrophobic analysis revealed two regions of loydroploobicity, one at the N terminus representing the signal peptide (predicted from amino acids 1 to 24), and a second between amino acids 266 and 288. The Tmpred program predicted that the latter region is likely to represent a transmembrane domain, and that BSMAP is expressed on cells as a type-I membrane-bound protein (Figure 4). Pattern and motif searching with existing databases unveiled a 37% identity with a recently identified protein of unknown function cloned from CD34⁺ hematopoietic stem/progenitor cells (Mao et al., 1998).

When using the BSMAP protein sequence to query dbEST, two murine and one rat EST (accession numbers W85136, AA444173 and AA942798 respectively) have been identified. These ESTs share significant homology with BSMAP both at the nucleic acid and amino acid level. Indeed, the predicted amino acid sequence of the rat EST (AA942798) showed 73 % identity with the BSMAP amino acid sequence with the murine ESTs showing 82 % identity for AA444173 and 70% identity for W85136. These ESTs are deemed to be cDNAs encoding the mouse and rat homologues of BSMAP.

### EXAMPLE 4: Tissue distribution of BSMAP-specific mRNA

### Materials and Methods: Northern blot analysis

The 1031 bp BSMAP cDNA fragment cloned in the plasmid pGEM-Teasy was excised by digestion with the restriction enzyme *Eco* RI and purified. The fragment was subsequently radiolabeled with [α-³²P]dCTP (220 TBq/mmol; Amersham France SA, Les Ulis, France) using the random priming method (Feinberg and Vogelstein, 1983). This radiolabeled fragment served as a probe to identify BSMAP mRNA transcripts in the human 12-lane multiple tissue Northern blot as well as in the human RNA Master blot (Clontech) Hybridizations were performed in ExpressHyb solution (Clontech). Hybridization and washing conditions were carried out according to the manufacturer's guidelines.

### Results

The source of origin of ESTs encoding BSMAP were first examined in order to determine in which tissues BSMAP mRNA was expressed (Table I, supra). Of the 45 ESTs identified, 35 ESTs were derived from brain suggesting a significant level of expression of BSMAP-speciFic mRNA in this tissue. The 3 ESTs derived human fetal tissue, the 3 derived from human synovial sarcoma and the 2 derived from mouse embryos also pointed to an expression of BSMAP mRNA in these tissues. The expression of BSMAP mRNA was analyzed by Northern blotting using polyadenylaled RNA derived from twelve different human tissues.
Amongst these tissues, a high level of BSMAP-specific mRNA expression was detected in brain, a signal with a molecular size corresponding to that of the cloned cDNA being readily detected following an exposure time of hours (Figure 6A), confirming the above observation that approximately 78 % of all BSMAP specific ESTs were derived from this source. Very low levels of expression were also detected in kidney, liver, small intestine and placenta following a prolonged exposure of the hybridized membrane. The distribution of BSMAP specific mRNA within the different regions of the CNS using a polyadenylated RNA dot blot was determined A high level of expression of BSMAP RNA was found in most regions of the CNS included as well as in fetal brain and the pituitary gland, relative to the other tissues present on the blot (Figure 6B) confirming the observation that BSMAP mRNA is both highly and preferentially expressed in the brain. The strongest expression was seen in whole brain, amygdala, occipital lobe and putamen, with lowest levels of brain-specific expression found in the spinal cord, medulla oblongata and substantia nigra (Figure 6B).

### EXAMPLE 5:

### Materials and Methods: Expression of BSMAP in transiently transfected COS1 cells

### 1- Transient Transfection.

The BSMAP cDNA cloned in pGEM-Teasy was re-amplified by PCR using the Advantage®-GC cDNA PCR kit with the following printers : 
5'-CCCAAGCTTACCATGGCT GCGGTGGCGCTG-3'(SEQ ID n° 12)
5'-CCGCTCGAGGGCCTACAGCTTGGTCAGGT-3'(SEQ ID n° 13).
 The amplified cDNA product was digested with the restriction enzymes *Hind* III and *Xho* I and cloned into the mammalian expression vector pCDNA3 (Invitrogen, Leek, The Netherlands). In order to generate a modified form of BSMAP containing a C-myc antibody recognition tag ((Evan et al., 1985); Calbiochem-Novabiochem Corp., San Diego, CA) within the predicted extracellular region of the protein, a portion of the BSMAP cDNA cloned in pGEM-Teasy was amplified by PCR using the Advantage®-GC cDNA PCR kit with the primers 5'-GAATTCACTAGTGATTTATGG-3' (SEQ ID n° 14) and 5'-CGCGGATCCCCAGATCCTCTTCTGAGATGAGTTTTGTT CGATCAGAACGGCTCTGTCAT-3'(SEQ ID n° 15). The resulting PCR fragment was digested with the restriction enzymes Spel and BamHI and recloned into the pGEM-Teasy/BSMAP cDNA construct digested with the aforementioned restriction enzymes. This modified BSMAP cDNA was subsequently reamplified by PCR using the Advantage®-GC cDNA PCR kit with the following primers : 
5'-CCCAAGCTTACCATGGCTGCGGTGGCGCTG-3'(SEQ ID n° 16)
5'-CCGCTCGAGGGC CTACAGCTTGGTCAGGT-3'(SEQ ID n° 17).
 The amplified cDNA product was digested with the restriction enzymes *Hind* III and *Xho* I and cloned into the mammalian expression vector pCDNA3. COS1 cells were transfected with the BSMAP expression constructs using the liposome transfection reagent FuGENE™ 6 (Boehringer Mannheim) following manufacturer's guidelines. In all cases, 1.5 µg of DNA and 6 µl of FuGENE™ 6 reagent were used per transfection.

### 2- Western Blot Analysis.

At 48 hours post-transfection, COS1 I cells and their supernatants were harvested. Cells were washed twice with 1 x PBS and resuspended in lysis buffer (25 mM Hepes, 1 % Triton-X-100, 1 mM EGTA, 100 mM NaCl, 10 % glycerol) at a concentration of 1 x 10⁷ cells/ml. Following a 30 minute incubation on ice, insoluble material was removed from the cell lysate by centrifugation for 10 minutes in a micocentrifuge. Cell supernatants were centrifuged sequentially for 10 minutes at 2 000 rpm with a 8160 rotor (Heraeus Instruments, Osterode, Germany) and for 30 minutes at 35 000 rpm with a SW60Ti rotor (Beckman Instruments Inc., Fullerton, CA) to remove cellular debris. Cell lysates and supernatants were diluted 1:1 in tris-glycine-SDS sample buffer containing 750 mM 2-Mercapthoethanol, Samples were heated to 95°C for 5 minutes, the proteins resolved on a 10 % SDS-polyacrylamide gel and electrotransferred to a nitrocellulose membrane (Burnette, 1981). Membranes were blocked in 1 x PBS containing 5 % dried milk and 0.1 % Tween-20 for 1 hour at room temperature followed by a subsequent 1 hour incubation in the same buffer containing the anti-C-myc tag monoclonal antibody at 5 µg/ml. Bound antibody was detected using horseradish-peroxidase labeled sheep anti-mouse Ab and ECL (Amersham France, SA) following the manufacturer's guidelines.

### Results.

In order to verify that the cDNA BSMAP was capable of encoding for a protein product, cDNAs encoding the full length ORF with or without an N-terminal C-myc antibody recognition tag were cloned into the mammalian expression vector pCDNA3 and used to transfect COS1 cells.
Western blot analysis revealed that the cDNA encoded a protein product of approximately 40 kDa, in accordance with the 38 kDa size predicted, which was detectable in the lysate of cells transfected with the cDNA encoding BSMAP containing the C-myc tag. As expected, no band was detected with the lysate of cells transfected with the cDNA encoding the native BSMAP. No signal was detected in the supernatant of cells transfected with cDNA encoding BSMAP and cells transfected with BSMAP containing the C-myc tag even following prolonged exposure, confirming that BSMAP was not soluble (Figure 7).

### REFERENCES

Ashworth, L.K., Batzer, M.A., Brandriff, B., Branscomb, E., de Jong, P., Garcia, E., Garnes, J.A., Gordon, L.A., Lamerdin, J.E., and Lennon, G. (1995). An integrated metric physical map of human chromosome 19. *Nat.Genet*. **11**:422-427.

Ausubel, F. M., Brent, R., Kingston, R. E., Moore, D. D., Seidman, J. G., Smith, J. A., and Struhl K. Eds. (1991). "Current Protocols in Molecular Biology," Wiley, New York.

Burnette, W.N. (1981). "Western blotting": electrophoretic transfer of proteins from sodium dodecyl sulfate-polyacrylamide gels to unmodified nitrocellulose and radiographic detection with antibody and radioiodinated protein A. *Anal.Biochem.* **112**: 195-203.

Elson, G.C., Graber, P., Losberger, C., Herren, S., Gretener, D., Menoud, L.N., Wells, T.N., Kosco-Vilbois, M.H., and Gauchat, J.F. (1998). Gytokine-like factor-1, a novel soluble protein, shares homology with members of the cytokine type I receptor family. *J.Immunol.* **161**:1371-1379.

Evan, G.I., Lewis, G.K., Ramsay, G., and Bishop, J.M. (1985). Isolation of monoclonal antibodies specific for human c-myc proto- oncogene product *Mol.Cell Biol.* **5**: 3610-3616.

Feinberg, A.P. and Vogelstein, B. (1983). A technique for radiolabeling DNA restriction endonuclease fragments to high specific activity. *Anal.Biochem.* **132**: 6-13.

Francke, U., Brown, M.S., and Goldstein, J.L. (1984). Assignment of the human gene for the low density lipoprotein receptor to chromosome 19: synteny of a receptor, a ligand, and a genetic disease. *Proc.Natl.Acad.Sci.U.S.A.* **81**: 2826-2830.

Gauchat, J.F., Aubry, J.P., Mazzei, G., Life, P., Jomotte, T., Elson, G., and Bonnefoy, J.Y. (1993). Human CD40-ligand: molecular cloning, cellular distribution and regulation of expression by factors controlling IgE production. *FEBS Lett.* **315**: 259-266.

Korenberg, J.R. and Rykowski, M.C. (1988). Human genome organization: Alu, lines, and the molecular structure of metaphase chromosome bands. *Cell* **53**: 391-400.

Kozak, M. (1989). The scanning model for translation: an update *J.Cell Biol*. **108**: 229-241.

Larsen, F., Gundersen, G., Lopez, R., and Prydz, H. (1992). CpG islands as gene markers in the human genome. *Genomics* **13**: 1095-1107.

Mao, M., Fu, G., Wu, J.S., Zhang, Q.H., Zhou, J., Kan, L.X., Huang, Q.H., He, K.L., Gu, B.W., Han, Z.G., Shen, Y., Gu, J., Yu, Y.P., Xu, S.H., Wang, Y.X., Chen, S.J., and Chen, Z. (1998). Identification of genes expressed in human CD34(+) hematopoietic stem/progenitor cells by expressed sequence tags and efficient full- length cDNA cloning. *Proc.Natl.Acad.Sci.U.S.A.* **95**: 8175-8180.

Okayama, H., Kawaichi, M., Brownstein, M., Lee, F., Yokota, T., and Arai, K. (1987). High-efficiency cloning of full-length cDNA; construction and screening of cDNA expression libraries for mammalian cells. *Methods Enzymol.* **154**: 3-28.

Pochon, S., Graber, P., Yeager, M., Jansen, K., Bernard, Alt., Aubry, J.P., and Bonnefoy, J.Y. (1992). Demonstration of a second ligand for the low affinity receptor for immunoglobulin E (CD23) using recombinant CD23 reconstituted into fluorescent liposomes *J.Exp.Med*. **176**: 389-397.

Schnyder, B., Lugli, S., Feng, N., Etter, H., Lutz, R.A., Ryffel, B., Sugamura, K., Wunderli-Allenspach, H., and Moser, R. (1996). lnterleukin-4 (IL-4) and IL-13 bind to a shared heterodimeric complex on endothelial cells mediating vascular cell adhesion moleculc-1 induction in the abscence of the common gamma chain. *Blood* **87**: 4286-4295.

Sokol, L., Luhovy, M., Guan, Y., Prehal, J.F., Semenza, G.L., and Prehal, J.T. (1995). Primary familial polycythemia: a frameshift mutation in the erythropoietin receptor gene and increased sensitivity of erythroid progenitors to erythropoietin. *Blood* **86**: 15-22.

Strittmatter, W.J. and Roses, A.D. (1995). Apolipoprotein E and Alzheimer disease. *Proc.Natl*.*Acad*.*Sci.U.S.A.* **92**: 4725-4727.

Thompson, J.D., Higgins. D.G., and Gibson, T.J. (1994). CLUSTAL W: improving the sensitivity of progressive multiple sequence alignment through sequence weighting, position-specific gap penalties and weight matrix choice. *Nucleic.Acid.Res*. **22**: 4673-4680.

Uberbacher, E.C. and Mural, R.J. (1991). Locating protein-coding regions in human DNA sequences by a multiple sensor-neural network approach. *Proc.Natl.Acad.Sci.U.S.A.* **88**: 11261-11265.

## Claims

**1.** A polynucleotide, comprising a sequence having at least 50% identity with the sequence of SEQ ID n° 1, coding for BSMAP.

**2.** A polynucleotide according to claim 1 comprising a sequence having at least 80% identity with the sequence of SEQ ID n°1, coding for the human BSMAP.

**3.** A polynucleotide according to one of claims 1 and 2, or a fragment thereof, which is chosen from the group consisting of DNA, CDNA, and RNA.

**4.** An oligonucleotide capable of hybridizing under stringent conditions a polynucleotide according to claim 3 for a use as a probe or a primer.

**5.** An oligonucleotide comprising at least 8 consecutive bases of a polynucleotide according to claim 4 for a use as a probe or a primer.

**6.** An oligonucleotide according to claim 5 which is a complementary antisense oligonucleotide to a sequence coding for BSMAP.

**7.** An oligonucleotide according to claim 5 which is labeled with a fluorescent or radioactive tag.

**8.** Use of an oligonucleotide according to one of claims 5 to 7 for the detection and/or the amplification of a sequence coding for BSMAP in a sample.

**9.** A method for detecting a sequence coding for BSMAP in a simple consisting of hybridizing the DNA contained in said sample with a oligonucleotide according to claim 7 and analyzing the signal.

**10.** A method for detecting a sequence coding for BSMAP in a sample comprising the steps consisting of :
c) Isolating and/or purifying the DNA extracted from a sample, or obtaining the cDNA corresponding to the RNA contained in a sample.
d) amplifying specifically said DNA or cDNA using oligonucleotides according to one of claims 4 to 6,
c) hybridizing the products obtained in step b) with a oligonucleotide according to claim 7 and analyzing the signal.

**11.** A kit for the detection and/or the amplification of a sequence coding for BSMAP in a sample comprising at least one oligonucleotide according to one of claims 5 to 7 and reagents necessary for detecting and/or amplifying.

**12.** A recombinant vector comprising a polynucleotide according to one of claims 1 to 3 for the cloning and/or the expression of said polynucleotide.

**13.** A recombinant vector according to claim 12 comprising at least one element allowing the efficient expression of said polynucleotide or fragment of polynucleotide in a host cell.

**14.** A host cell transformed by a vector according to one of claims 12 and 13.

**15.** A process for preparing a BSMAP polypeptide, or fragment thereof, wherein cells according to claim 14 are cultured in conditions allowing an efficient production of said polypeptide.

**16.** A process for preparing a BSMAP polypeptide using a vector according to claim 13.

**16.** A polypeptide obtainable by a process according to one of claims 15 and 16.

**17.** A polypeptide comprising an amino acid sequence having at least 70 % identity with the sequence of SEQ. ID n°2.

**18.** A polypeptide which is chosen from the group consisting of a homologue, a derivative and a fragment of a polypeptide according to claim 17.

**19.** A monoclonal or polyclonal antibody which is capable of binding selectively to a polypeptide according to one of claims 16 to 18.

**20.** An antibody according to claim 19 which is labeled with a radioactive or a fluorescent tag, or which is linked to an enzyme reacting with a chromogene, a fluorigene or luminescent substrate.

**21.** A monoclonal antibody according to claim 19 which is capable of modulating BSMAP activity.

**22.** A kit for detecting a polypeptide according to one of claims 16 to 18 in a sample comprising a monoclonal or polyclonal antibody according to one of claims 19 and 20, a buffer favoring the polypeptide-antibody complex formation, and optionally agents allowing the detection of said complex.

**23.** A process for screening agonist or antagonist compounds comprising the step consisting of testing the affinity and/or the effect of said compounds on a polypeptide according to one of claims 16 to 18.

**24.** A process according to claim 23 wherein said compounds are tested on a host cell according to claim 14.

**25.** An agonist or antagonist compound obtainable from a process according to one of claims 23 and 24.

**26.** A compound according to claim 25 which is selected from chemicals, antibodies, peptides, and peptide derivatives.

**27.** A pharmaceutical composition comprising a compound according to claim 26 and a pharmaceutically acceptable carrier.

**28.** Use of a compound according to claim 26 for preparing a medicament for treating and/or preventing CNS disorders.

**29.** Use according to claim 28 for treating and/or preventing depression, dyslexia, dystonia, eating disorders, epilepsy, migrane, headache, panic disorder, schizophrenia, obsessive and compulsive disorders.

**30.** Use of a compound according to claim 26 for preparing a medicament for treating and/or preventing CNS diseases.

**31.** Use according to claim 30 for treating and/or preventing amotrophic lateral sclerosis, multiple sclerosis, Alzheimer's disease, brain tumors, Hunlington's disease, Parkinson's disease and stroke.


LISTAGE DE SEQUENCE

<110> PIERRE FABRE

<120> GENE BSMAP

<130> D17990

<140>
<141>

<160> 17

<170> PatentIn Vers. 2.0

<210> 1
<211> 1557
<212> ADN
<213> Homo sapiens

<220>

<400> 1
atggctgcgg tggcgctgat gccaccgccg ctgctgctgc tgctgctgtt ggcgtcgccg 60
cccgccgcct ccgcgccgtc cgcccgcgat cccttcgccc cccagctcgg ggacacgcag 120
aactgccagc tgcggtgccg cgaccgcgac ctcggcccgc agccctcgca ggcggggctg 180
gagggcgcct ccgagtctcc ctatgacaga gccgttctga tcagcgcttg cgagcgtggc 240
tgccgcctct tctccatctg ccgatttgtg gccagaagct ccaagcccaa tgccacccaa 300
actgagtgtg aagcagcctg cgtggaagcc tatgtgaagg aggcagagca gcaggcctgt 360
agccacggct gctggagcca gcccgcggag cctgagccgg agcagaagag aaaggtcctg 420
gaggctccaa gtggggccct ctccctcttg gacttgtttt ccaccctctg caatgacctt 480
gtcaactcag cccagggatt tgtctcctcc acctggacat actacttgca gactgacaat 540
gggaaagtgg tggtgtttca gactcagccc atagtggaga gcctcggctt ccaggggggc 600
cgtctgcagc gcgtggaggt gacctggcga ggctcccacc ctgaagccct ggaggtgcac 660
gtggaccctg taggccccct ggacaaggtg aggaaggcca agatccgagt caagaccagc 720
agcaaggcca aggtggagtc tgaagagcca caggacaatg acttcctcag ttgcatgtcc 780
cggcgctcgg gtctgcctcg ctggatcctg gcctgctgcc tcttcctctc cgtgctggtg 840
atgctgtggc tgagctgctc caccctggtg accgcgcctg gccagcacct caagttccag 900
cctctgaccc tggagcagca caagggcttc atgatggagc ccgattggcc cctgtacccg 960
ccgccgtccc acgcctgtga ggacagccta ccaccctaca agctgaagct ggacctgacc 1020
aagctgtagg cctccactgg ccccatcact gccaactgca gggggcccct cgggcctcac 1080
ttgccctgag cccaggagtc caagggcagg gtgggtccag ccttgagccc ctccaccccc 1140
aaatccttcc tctcctccca gtcccacccc ttgccccacg gagtcctggg gacgcagtgc 1200
cccagctggg aagagggcgg gatcgggcac tggttcctcc ttgtccccgc tttcttgggg 1260
gcttgctact ttttgtcttc tattgtgtgg ctttctgagt atttgaaccc cagtcctgtg 1320
tcaccttcct ttttccttct atgtcccctc tctgcggggg gggcgctgag gctgaggggg 1380
agctgcgtct tgctagggct tcccccttct ccccatcccg gtctccagaa cccagctttt 1440
gagagacagg gtgtgggcat ctccatgccc ctataaagcg tgcctggggc ttgtctgggg 1500
ctggggagga ataaaccatg tatataaaag aaaaaaaaaa aaaaaaaaaa aaaaaaa 1557

<210> 2
<211> 342
<212> PRT
<213> Homo sapiens

<220>

<400> 2
Met Ala Ala Val Ala Leu Met Pro Pro Pro Leu Leu Leu Leu Leu Leu
  1               5                  10                  15

Leu Ala Ser Pro Pro Ala Ala Ser Ala Pro Ser Ala Arg Asp Pro Phe
            20                  25                  30

Ala Pro Gln Leu Gly Asp Thr Gln Asn Cys Gln Leu Arg Cys Arg Asp
        35                  40                  45

Arg Asp Leu Gly Pro Gln Pro Ser Gln Ala Gly Leu Glu Gly Ala Ser
    50                  55                  60

Glu Ser Pro Tyr Asp Arg Ala Val Leu Ile Ser Ala Cys Glu Arg Gly
65                  70                  75                  80

Cys Arg Leu Phe Ser Ile Cys Arg Phe Val Ala Arg Ser Ser Lys Pro
                85                  90                  95

Asn Ala Thr Gln Thr Glu Cys Glu Ala Ala Cys Val Glu Ala Tyr Val
            100                 105                 110

Lys Glu Ala Glu Gln Gln Ala Cys Ser His Gly Cys Trp Ser Gln Pro
        115                 120                 125

Ala Glu Pro Glu Pro Glu Gln Lys Arg Lys Val Leu Glu Ala Pro Ser
    130                 135                 140

Gly Ala Leu Ser Leu Leu Asp Leu Phe Ser Thr Leu Cys Asn Asp Leu
145                 150                 155                 160

Val Asn Ser Ala Gln Gly Phe Val Ser Ser Thr Trp Thr Tyr Tyr Leu
                165                 170                 175

Gln Thr Asp Asn Gly Lys Val Val Val Phe Gln Thr Gln Pro Ile Val
            180                 185                 190

Glu Ser Leu Gly Phe Gln Gly Gly Arg Leu Gln Arg Val Glu Val Thr
        195                 200                 205

Trp Arg Gly Ser His Pro Glu Ala Leu Glu Val His Val Asp Pro Val
    210                 215                 220

Gly Pro Leu Asp Lys Val Arg Lys Ala Lys Ile Arg Val Lys Thr Ser
225                 230                 235                 240

Ser Lys Ala Lys Val Glu Ser Glu Glu Pro Gln Asp Asn Asp Phe Leu
                245                 250                 255

Ser Cys Met Ser Arg Arg Ser Gly Leu Pro Arg Trp Ile Leu Ala Cys
            260                 265                 270

Cys Leu Phe Leu Ser Val Leu Val Met Leu Trp Leu Ser Cys Ser Thr
        275                 280                 285

Leu Val Thr Ala Pro Gly Gln His Leu Lys Phe Gln Pro Leu Thr Leu
    290                 295                 300

Glu Gln His Lys Gly Phe Met Met Glu Pro Asp Trp Pro Leu Tyr Pro
305                 310                 315                 320

Pro Pro Ser His Ala Cys Glu Asp Ser Leu Pro Pro Tyr Lys Leu Lys
                325                 330                 335

Leu Asp Leu Thr Lys Leu

            340

<210> 3
<211> 20
<212> ADN
<213> Artificial Sequence

<220>

<400> 3
tgccgcctct tctccatctg                                                    20

<210> 4
<211> 20
<212> ADN
<213> Artificial Sequence

<220>

<400> 4
agccgaggct ctccactatg                                                    20

<210> 5
<211> 21
<212> ADN
<213> Artificial Sequence

<220>

<400> 5
ggcgacgagg cccagagcaa g                                                  21

<210> 6
<211> 20
<212> ADN
<213> Artificial Sequence

<220>

<400> 6
cgatttcccg ctcggccgtg                                                    20

<210> 7
<211> 20
<212> ADN
<213> Artificial Sequence

<220>

<400> 7
cgatttcccg ctcggccgtg                                                    20

<210> 8
<211> 20
<212> ADN
<213> Artificial Sequence

<220>

<400> 8

tgccgcctct tctccatctg                                                    20

<210> 9
<211> 26
<212> ADN
<213> Artificial Sequence

<220>

<400> 9
ttgtctcctc cacctggaca tactac                                             26

<210> 10
<211> 20
<212> ADN
<213> Artificial Sequence

<220>

<400> 10
atggctgcgg tggcgctgat                                                    20

<210> 11
<211> 20
<212> ADN
<213> Artificial Sequence

<220>

<400> 11
ggcctacagc ttggtcaggt                                                    20

<210> 12
<211> 30
<212> ADN
<213> Artificial Sequence

<220>

<400> 12
cccaagctta ccatggctgc ggtggcgctg                                         30

<210> 13
<211> 29
<212> ADN
<213> Artificial Sequence

<220>

<400> 13
ccgctcgagg gcctacagct tggtcaggt                                          29

<210> 14
<211> 21
<212> ADN
<213> Artificial Sequence

<220>

<400> 14
gaattcacta gtgatttatg g                                                  21

<210> 15
<211> 60
<212> ADN
<213> Artificial Sequence

<220>

<400> 15
cgcggatccc cagatcctct tctgagatga gtttttgttc gatcagaacg gctctgtcat 60

<210> 16
<211> 30
<212> ADN
<213> Artificial Sequence

<220>

<400> 16
cccaagctta ccatggctgc ggtggcgctg 30

<210> 17
<211> 29
<212> ADN
<213> Artificial Sequence

<220>

<400> 17
ccgctcgagg gcctacagct tggtcaggt 29